# EUROPEAN PATENT APPLICATION

(11) **EP 3 284 368 A1**
(43) Date of publication of application: **21.02.2018**
(21) Application number: 17182773.6
(22) Date of filing: 24.07.2017
(51) Int. Cl.: A45D 33/34

(54) **ANTIBACTERIAL PUFF FOR COSMETICS**

(30) Priority: 28.07.2016 KR 20160095971
(71) Applicant: DMCOSMETICS CO., LTD., Incheon 21694 (KR)
(72) Inventor: CHOI, Da Hee, Incheon (KR)
(74) Representative: Grünecker Patent- und Rechtsanwälte PartG mbB

(57) **Abstract**

The present invention relates to an antibacterial puff for cosmetics in which an antibacterial agent is included in a foam composed of polyurethane and the like, and more particularly, to an antibacterial puff which reduces excessive consumption of a cosmetic material caused by absorption of a puff, which is a disadvantage of an existing puff, and includes an antibacterial material capable of suppressing proliferation of microorganisms.

## Description

### CROSS-REFERENCE TO RELATED APPLICATION

This application claims priority to and the benefit of Korean Patent Application No. 10-2016-0095971, filed on Jul 28, 2016, the disclosure of which is incorporated herein by reference in its entirety.

### BACKGROUND

### 1. Field of the Invention

The present invention relates to an antibacterial puff for cosmetics, and more particularly, to an antibacterial puff for cosmetics, which improves the waste of a cosmetic material caused by excessive absorption, which is a disadvantage of a puff for liquid cosmetics and is composed of urethane (PU) foam and the like containing an antibacterial agent which is developed to suppress proliferation of microorganisms.

### 2. Discussion of Related Art

Generally, liquid cosmetic materials in makeup refer to cosmetics for base makeup which decorate the face so that skin tone becomes natural beige and the like, and cosmetic materials as the content are mainly prepared in a form of a solution (liquid material).

The liquid cosmetic materials in makeup were aliquoted and applied to the face by hand in the past, and therefore the hands need to be wiped with a tissue or washed after makeup.

In order to eliminate this inconvenience, a puff was developed to avoid using the hand. Conventional puffs have been prepared with porous materials such as nitrile butadiene rubber (NBR), styrene butadiene rubber (SBR), natural rubber, flocking, Rubycell (wet urethane), cotton velour, polyvinyl alcohol (PVA) and the like.

The puff is convenient in use because an appropriate amount of a liquid cosmetic material can be aliquoted from a cosmetic container using a puff and applied to the face. However, the waste of a cosmetic material is caused by a puff excessively absorbing a cosmetic material and the period of use of cosmetics is shortened, and therefore an economic burden and inconvenience are imparted by having to buy cosmetics frequently.

In addition, a puff directly comes in contact with the skin when a cosmetic material is applied, and thus, a cosmetic material is likely to be contaminated with microorganisms because a puff contaminated with microorganisms comes in contact with a cosmetic material in a container again. The face provides good conditions for proliferation of microorganisms due to sebum and sweat secreted in the skin, dust in the atmosphere, touching the face with a hand contaminated with microorganisms and the like. When a cosmetic material is aliquoted and applied to such a face using a puff, microorganisms may be transferred to the puff, and the puff also creates good conditions for proliferation of microorganisms due to the supply of moisture and nourishment by a cosmetic material and thermal retention of a container. Since a cosmetic material is a liquid including water, a cosmetic container with temperature, moisture and nutrient requirements, an enclosed handbag that stores a cosmetic container, or a warm indoor space where a dressing table on which cosmetic products are placed is positioned creates a good temperature condition for proliferation of microorganisms due to thermal retention. In addition to a puff contaminated with microorganisms, a cosmetic material contaminated in a container results in skin trouble caused by microorganisms.

As such, a conventional puff used to apply a liquid cosmetic material to the face causes not only waste of a cosmetic material by excessively absorbing a cosmetic material, but also contamination of a cosmetic material as well as the puff itself with microorganisms by directly coming in contact with the skin. Therefore, it is necessary to develop an antibacterial puff for cosmetics which can reduce the waste of a cosmetic material and suppress the proliferation of microorganisms.

### [Prior-Art Documents]

### [Patent Documents]

(Patent Document 1) 1. Korea Utility Model Registration Application Publication No. 20-2009-0005978 (June 17, 2009)

### SUMMARY OF THE INVENTION

It is an object of the present invention to provide an antibacterial puff for cosmetics, which is capable of improving the waste of a cosmetic material by forming a barrier layer having a waterproof film structure for blocking penetration of a cosmetic material into an intermediate layer and designing a puff in such a way that an appropriate amount of a cosmetic material is applied to a lower layer in order to prevent the waste of a cosmetic material caused by penetration of a cosmetic material from a lower layer coming in contact with the skin to an intermediate layer in a conventional puff, and includes silver glass as an antibacterial component in a lower layer thereof for suppressing proliferation of microorganisms.

According to an embodiment of the present invention, there is provided an antibacterial puff for cosmetics which includes a lower layer configured to come in contact with a cosmetic material to absorb the cosmetic material and containing an antibacterial component; a barrier layer provided on the lower layer and having a waterproof film structure for blocking penetration of the cosmetic material; a first adhesive layer provided on the barrier layer; an intermediate layer provided on the first adhesive layer; a second adhesive layer provided on the intermediate layer; and an upper layer provided on the second adhesive layer.

In the antibacterial puff for cosmetics according to the present invention, the barrier layer may be formed by using a waterproof adhesive so as to form a waterproof film.

In the antibacterial puff for cosmetics according to the present invention, the antibacterial component may be silver glass containing silver at 1 to 3 wt%, and may be contained at 0.1 to 3 wt% in the lower layer.

In the antibacterial puff for cosmetics according to the present invention, the lower layer may have a thickness of 0.3 to 3 mm, and the barrier layer may have a thickness of 0.01 to 0.1 mm.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 is a partially-cut perspective view of a puff for cosmetics according to the present invention;
FIG. 2 is a cross-sectional view of a puff for cosmetics according to the present invention;
FIG. 3 is an exploded perspective view of a puff for cosmetics according to the present invention;
FIG. 4 shows electron microscope images of an untreated puff and an antibacterial puff; and
FIG. 5 shows images illustrating antibacterial test results of antibacterial-treated and untreated samples with respect to *E. coli.*

### DETAILED DESCRIPTION OF EXEMPLARY EMBODIMENTS

Hereinafter, the present invention will be described in detail with reference to the accompanying drawings.

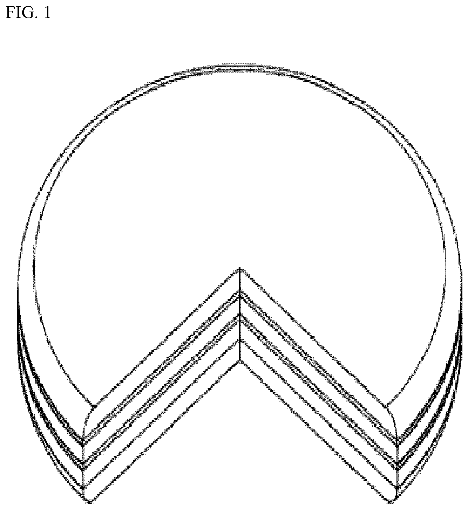

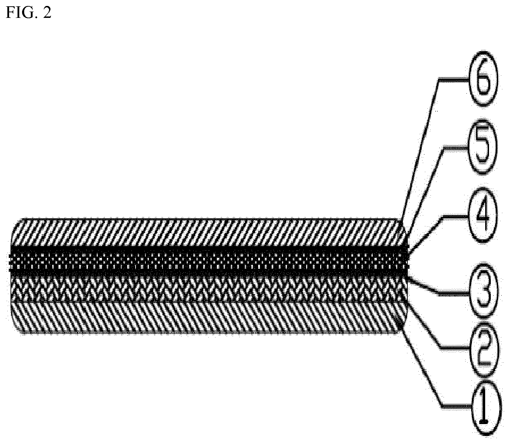

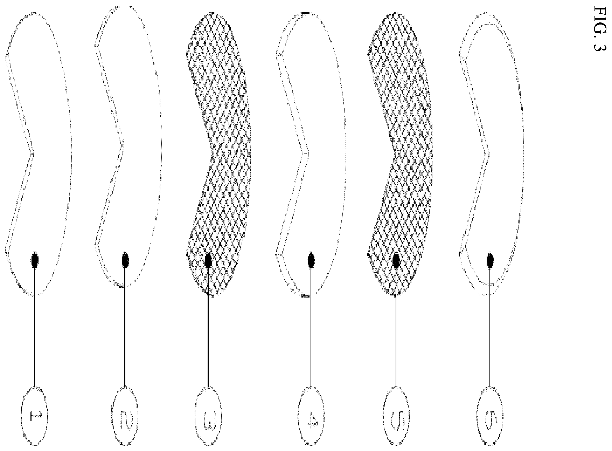

FIG. 1 is a partially-cut perspective view of a puff for cosmetics according to the present invention, FIG. 2 is a cross-sectional view of a puff for cosmetics according to the present invention, and FIG. 3 is an exploded perspective view of a puff for cosmetics according to the present invention. A puff for cosmetics according to the present invention may be composed of a lower layer 1, a barrier layer 2, a first adhesive layer 3, an intermediate layer 4, a second adhesive layer 5, and an upper layer 6, which are laminated sequentially from the bottom.

The lower layer 1 comes in contact with cosmetic contents provided in a cosmetic packaging material. The barrier layer 2 forms a waterproof film to block absorption of a cosmetic material into the intermediate layer 4. The first adhesive layer 3 is provided on the barrier layer 2 to attach the intermediate layer 4 on the barrier layer 2. The intermediate layer 4 is attached on the barrier layer 2 by the first adhesive layer 3. The second adhesive layer 5 is provided on the intermediate layer 4 to attach the upper layer 6 on the intermediate layer 4. The upper layer 6 is attached on the intermediate layer 4 by the second adhesive layer 5.

The cosmetic material may be a liquid-phase cosmetic material or a powder-phase cosmetic material. The cosmetic material may be applied to other types of cosmetic materials such as gel-phase, paste-phase and cake-phase cosmetic materials as well as the liquid-phase cosmetic material and powder-phase cosmetic material.

The lower layer 1 is composed of polyurethane and the like with an open cell structure, which is a mesh structure, so that cosmetic contents can be absorbed, and thus the applicability and spreadability of cosmetic contents may be improved. The lower layer 1 may have a thickness of 0.3 to 3 mm, and it is possible to minimize the volume of an absorbed cosmetic material within this range of thickness.

The lower layer 1 may be composed of a porous material with an open cell structure so that the cosmetic material may be uniformly adhered without agglomeration. The porous material may be any one or more selected from nitrile butadiene rubber (NBR), styrene butadiene rubber (SBR), natural rubber, flocking, Rubycell (wet urethane), cotton velour, polyvinyl alcohol (PVA) and urethane (PU) foam. However, the lower layer 1 is not limited to the above-described porous materials, that is, a lower layer 1 which is composed of other materials capable of applying the cosmetic material without agglomeration may be applied in various modified examples.

The lower layer 1 of the puff coming in contact with the skin may suppress proliferation of microorganisms in a puff and a cosmetic material which the puff absorbs by containing an antibacterial component in a foam sheet such as polyurethane and the like. As necessary, in addition to the lower layer 1, at least one layer of the other layers such as the barrier layer 2, the first adhesive layer 3, the intermediate layer 4, the second adhesive layer 5 and the upper layer 6 may also contain an antibacterial component.

The antibacterial component may be silver glass (CAS No 65997-17-3) containing silver at 1 to 3 wt%. For example, silver glass containing silver at 1.8 wt% may be used. When silver glass is used, antibacterial activity is effectively imparted to a puff for cosmetics.

The lower layer 1 may contain silver glass at 0.1 to 3 wt%. When an amount of an antibacterial component is too small, sufficient antibacterial activity may be not exhibited, and when an amount thereof is too large, other properties may be degraded. For example, the lower layer 1 may be composed of a base material at 80 wt% and an antibacterial masterbatch at 20 wt%, the antibacterial masterbatch may be composed of a base material at 90 wt% and silver glass at 10 wt%, and the silver glass may contain silver at 1.8 wt%. As another example, the lower layer 1 may be composed of a base material at 99 wt% and silver glass at 1 wt%, and the silver glass may contain silver at 1.8 wt%. Within the above ranges of content, antibacterial activity of 99.96% or more with respect to *Staphylococcus aureus* (*S. aureus*) and antibacterial activity of 99.36% or more with respect to *E. coli* may be ensured.

The barrier layer 2 has a waterproof film structure to block absorption of cosmetic contents into the intermediate layer 4, and thus the waste of cosmetic contents may be reduced. The barrier layer 2 may have a thickness of 0.01 to 0.1 mm, and it is possible to block absorption of a cosmetic material into the intermediate layer 4 within this range of thickness.

The barrier layer 2 may use an adhesive to form a waterproof film for blocking absorption of a cosmetic material. For example, the barrier layer 2 may be formed by using a waterproof adhesive. The waterproof adhesive may be a silicone-based, urethane-based, epoxy-based, or latex-based (SBR latex and the like) waterproof adhesive.

Preferably, an epoxy-based waterproof adhesive composition including an epoxy resin and an amine-based curing agent is used. The epoxy resin may be at least one resin selected from an epoxy resin having a glycidyl amino group derived from m-xylylenediamine, an epoxy resin having a glycidyl amino group derived from 1,3-bis(aminomethyl)cyclohexane, an epoxy resin having a glycidyl amino group derived from diaminodiphenylmethane, an epoxy resin having a glycidyl amino group and/or a glycidyloxy group derived from p-aminophenol, an epoxy resin having a glycidyloxy group derived from bisphenol A, an epoxy resin having a glycidyloxy group derived from bisphenol F, an epoxy resin having a glycidyloxy group derived from phenol novolac, and an epoxy resin having a glycidyloxy group derived from resorcinol, and is preferably an epoxy resin having a glycidyl amino group derived from m-xylylenediamine.

The amine-based curing agent may be a reaction product of (A)+(B)+(D) or a reaction product of (A)+(B)+(C)+(D) among (A) m-xylylenediamine or p-xylylenediamine, (B) a multifunctional compound having at least one acyl group which may form an amide group site by reaction with a polyamine and may also form an oligomer (e.g., acrylic acid, methacrylic acid and/or a derivative thereof), (C) a monocarboxylic acid having 1 to 8 carbon atoms and/or a derivative thereof (e.g., formic acid, acetic acid, propionic acid, butyric acid, lactic acid, glycolic acid, benzoic acid and/or a derivative thereof), (D) a functional compound having at least one carbonate site which forms a carbamate site by reaction with a polyamine (e.g., ethylene carbonate and/or propylene carbonate). For example, the amine-based curing agent may be a reaction product of (a) m-xylylenediamine, (b) acrylic acid, methacrylic acid and/or a derivative thereof, and (d) ethylene carbonate, propylene carbonate or trimethylene carbonate. Here, a molar ratio of (a):(b):(d) may be 1:0.7 to 0.95:0.1 to 0.7.

A mixed ratio of the epoxy resin and amine-based curing agent may be 0.5 to 5.0 as an equivalent ratio of active hydrogen of the amine-based curing agent to an epoxy group of the epoxy resin (active hydrogen/epoxy group). The oxygen transmission coefficient of a cured product obtained by curing an epoxy-based waterproof adhesive composition may be 1.0 ml·mm/m²·day·MPa or less (23 °C, 60% RH).

The first adhesive layer 3 serves to immovably fix the barrier layer 2 and the intermediate layer 4 using an adhesive. The first adhesive layer 3 may be formed by using a common adhesive, for example, an acrylic, urethane-based, epoxy-based adhesive or the like, and may also be formed by using the same adhesive as that of the barrier layer 2.

The intermediate layer 4 as a cushion layer holds air so that the cosmetic material is gently adhered to the skin. The intermediate layer 4 may include a foam prepared with one or more selected from the group consisting of acrylonitrile butadiene rubber (NBR), styrene butadiene rubber (SBR), natural rubber (NR), polyvinyl chloride, polyethylene, ethylene-vinyl acetate butyl rubber (EVA), latex, silicone, styrene-isoprene-styrene (SIS), styrene-ethylene-butylene-styrene (SEBS), polyvinyl alcohol (PVA), nitrile rubber, butyl rubber, chloroprene rubber (CR), and urethane (PU) foam. In another aspect of the present invention, silicone foam may include foam prepared with a silicone-based elastomer. A material of the intermediate layer 4 is not limited to foam with an open cell structure, and various changes may be made.

The second adhesive layer 5 serves to immovably fix the intermediate layer 4 and the upper layer 6 using an adhesive. The second adhesive layer 5 may be formed by using a common adhesive, for example, an acrylic, urethane-based, epoxy-based adhesive or the like, and may also be formed by using the same adhesive as that of the first adhesive layer 3 or the barrier layer 2.

The upper layer 6 serves to maintain a shape of a puff and fix a ribbon. The upper layer 6 may be composed of urethane foam and the like.

A method of preparing the antibacterial puff for cosmetics according to the present invention may include preparing the lower layer 1 coming in contact with a cosmetic material; forming the barrier layer 2 on the lower layer 1; forming the first adhesive layer 3 on the barrier layer 2; attaching the intermediate layer 4 on the barrier layer 2 by the first adhesive layer 3; forming the second adhesive layer 5 on the intermediate layer 4; and attaching the upper layer 6 on the intermediate layer 4 by the second adhesive layer 5. Here, the barrier layer 2 may have a waterproof film structure so that content does not penetrate the barrier layer 2, and the lower layer 1 may have a structure including silver glass as an antibacterial component.

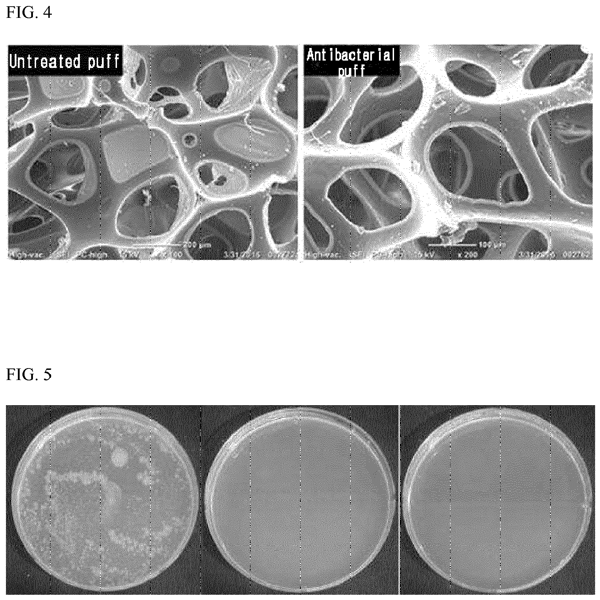

Table 1 shows an antibacterial test result of a puff according to a water immersion processing [classification 1], and Table 2 shows an antibacterial test result of a puff according to a light exposure processing [classification 1]. FIG. 4 shows electron microscope images of an untreated puff which does not include silver glass as an antibacterial material and an antibacterial puff which includes silver glass as an antibacterial material. The antibacterial test was performed according to a test method, a (version in fiscal year 2012) shake method.

**[Table 1]**

| Strain | Sample name | Initial number of bacteria | After 24 hours | Reduction rate (%) |
|---|---|---|---|---|
| *S. aureus* (*Staphylococcus aureus*) | Antibacterial Treated | 1.8 x 10⁹ | 4.8 x 10⁴ | 99.997% |
| | Control | | 2.6 x 10⁹ | |
| *E. coli* (*Escherichia coli*) | Antibacterial Treated | 1.2 x 10⁴ | 27 | 99.771% |
| | Control | | 7.3 x 10⁸ | |

**[Table 2]**

| Strain | Sample name | Initial number of bacteria | After 24 hours | Reduction rate (%) |
|---|---|---|---|---|
| *S. aureus* | Antibacterial Treated | 4.8 x 10⁶ | 1.8 x 10³ | 99.963% |
| | Control | | 2.6 x 10⁹ | |
| *E. coli* | Antibacterial Treated | 1.6 x 10³ | <10 | 99.369% |
| | Control | | 7.3 x 10⁸ | |

Table 3 shows the comparison of antibacterial test results after an antibacterial-treated sample was washed with a neutral detergent 20 times and after an antibacterial-treated sample was subjected to friction washing (washed by rubbing it). FIG. 5 shows images illustrating antibacterial test results of antibacterial-treated and untreated samples with respect to *E. coli.* A left image illustrates an untreated sample (control), a middle image illustrates a sample which was washed with a neutral detergent 20 times, and a right image illustrates a sample which was subjected to friction washing.

**[Table 3]**

| Strain | Sample name | Initial number of bacteria | After 24 hours | Reduction rate (%) |
|---|---|---|---|---|
| *E. coli* | Washing with neutral detergent 20 times | 2.0 x 10⁵ | <10 | 100% |
| | Control | | 2.9 x 10⁵ | |
| *E. coli* | Friction washing | 2.0 x 10⁵ | <10 | 100% |
| | Control | | 2.9 x 10⁵ | |

The antibacterial puff for cosmetics according to the present invention is not limited to the above-described examples, and various modifications can be made by those skilled in the art without departing from the technical scope of the following claims, thus the present invention covers all such modifications provided they are within the scope of the appended claims and their equivalents.

A puff according to the present invention can improve the waste of a cosmetic material because a cosmetic material is not absorbed into an intermediate layer by forming a barrier layer for blocking penetration of a cosmetic material into an intermediate layer after a cosmetic material penetrates a lower layer, and can suppress proliferation of microorganisms by including an antibacterial component. That is, the present invention provides an antibacterial puff for cosmetics, which improves the waste of a cosmetic material by minimizing the volume of an absorbed cosmetic material and has antibacterial activity.

**[List of Reference Numerals]**

| | |
|---|---|
| 1: lower layer | 2: barrier layer |
| 3: first adhesive layer | 4: intermediate layer |
| 5: second adhesive layer | 6: upper layer |

## Claims

1. An antibacterial puff for cosmetics comprising:
a lower layer configured to come in contact with a cosmetic material to absorb the cosmetic material and containing an antibacterial component;
a barrier layer provided on the lower layer and having a waterproof film structure for blocking penetration of the cosmetic material;
a first adhesive layer provided on the barrier layer;
an intermediate layer provided on the first adhesive layer;
a second adhesive layer provided on the intermediate layer; and
an upper layer provided on the second adhesive layer.

2. The antibacterial puff for cosmetics according to claim 1, wherein the barrier layer is formed by using a waterproof adhesive.

3. The antibacterial puff for cosmetics according to claim 1, wherein the antibacterial component is silver glass containing silver at 1 to 3 wt% and is contained at 0.1 to 3 wt% in the lower layer.

4. The antibacterial puff for cosmetics according to claim 1, wherein the lower layer has a thickness of 0.3 to 3 mm and the barrier layer has a thickness of 0.01 to 0.1 mm.
